# EUROPEAN PATENT APPLICATION

(11) **EP 4 835 460 A1**
(43) Date of publication of application: **04.11.2026**
(21) Application number: 24913730.8
(22) Date of filing: 27.12.2024
(51) Int. Cl.: G01N 27/327, G01N 33/483, G01N 33/66

(54) **BIOSENSOR FOR DETECTING GLUCOSE IN BODY FLUID, MANUFACTURING METHOD THEREOF, AND SYSTEM FOR MEASURING CONCENTRATION OF GLUCOSE IN BODY FLUID USING SAME**

(30) Priority: 28.12.2023 KR 20230194356; 27.11.2024 KR 20240172354
(71) Applicant: Dongwoon Anatech Co., Ltd., Seoul 06716 (KR)
(72) Inventor: IM, Eun Hye, Incheon 21997 (KR); KIM, Mi Rim, Suwon-si, Gyeonggi-do 16508 (KR); KIM, Mi Hee, Incheon 22019 (KR); CHOE, A Jeong, Seoul 05824 (KR); JANG, In Seop, Seoul 02873 (KR); AN, Jinho, Incheon 21982 (KR)
(74) Representative: Cabinet Beau de Loménie
(86) International application number: PCT/KR2024/021364
(87) International publication number: WO 2025/143926

(57) **Abstract**

Provided is a biosensor for detecting glucose in a body fluid using a measuring device, the biosensor comprising: a substrate; a plurality of electrodes positioned on the substrate; and a reagent layer, wherein the reagent layer comprises: an oxidoreductase; and an electron transfer mediator comprising a ruthenium compound and Neutral red.

## Description

### TECHNICAL FIELD

One embodiment relates to a biosensor for detecting glucose in a body fluid and a method of manufacturing the same. In addition, another embodiment relates to a system for measuring a concentration of glucose in a body fluid using the biosensor.

### BACKGROUND

Quantitatively or qualitatively analyzing an analyte present in a body fluid is chemically and clinically important. For example, measuring blood glucose in blood for a diabetic patient or measuring cholesterol, which is a cause of various adult diseases, is a representative example.

In an electrochemical biosensor using enzyme activity (for example, a glucose sensor, a uric acid sensor, a protein sensor, a DNA sensor, a sucrose sensor, a GOT (Glutamate-Oxaloacetate Transaminase) or GPT (Glutamate-Pyruvate Transaminase) sensor, or the like), measuring the enzyme activity more quickly and reproducibly by reacting with a specimen (for example, a specific substance present in a body fluid including saliva or blood) is a very important matter.

Accordingly, the biosensor comprises a reagent layer for causing a chemical change or a physical change by reacting with the specimen, and an electrode structure for measuring the change as a current.

Since a specimen measurement principle of such a biosensor estimates a concentration of a specimen (for example, glucose) in a body fluid using a current generated in response to the specimen reacting with the reagent layer, a current sensing technique for improving accuracy of specimen measurement is required.

### SUMMARY

### TECHNICAL PROBLEM

To implement current sensing for improving accuracy of specimen measurement, one embodiment configures a reagent layer with an oxidoreductase and an electron transfer mediator comprising a ruthenium compound and Neutral red.

In this case, one embodiment proposes a reagent layer that adjusts a concentration of the electron transfer mediator so as to satisfy a condition that an increase in current corresponding to an increase in the concentration of the specimen in the body fluid is to be equal to or greater than a rate of increase at which sensing is possible, and a condition that the current is to be equal to or greater than a threshold value at which sensing is possible with respect to a reference concentration of the specimen in the body fluid.

However, the technical problems to be solved by the present invention are not limited to the above-described problem, and may be variously expanded within a scope that does not depart from the technical idea and area of the present invention.

### TECHNICAL SOLUTION

According to one embodiment, a reagent layer provided on an electrode structure of a biosensor may comprise an oxidoreductase, and an electron transfer mediator comprising a ruthenium compound and Neutral red.

According to one aspect, the concentration of the Neutral red may be characterized in that it is adjusted such that a rate at which a current generated in response to a specimen in the body fluid being subjected to an oxidation-reduction reaction with the oxidoreductase increases in correspondence with an increase in the concentration of the specimen is equal to or greater than a predetermined rate of increase.

According to another aspect, the concentration of the Neutral red may be characterized in that it is adjusted such that a current generated in response to a specimen being subjected to an oxidation-reduction reaction with the oxidoreductase is equal to or greater than a predetermined threshold value.

According to still another aspect, the ruthenium compound may be characterized in that it comprises Hexaammineruthenium trichloride.

According to still another aspect, FAD-GDH (Flavin Adenine Dinucleotide-dependent Glucose Dehydrogenase) may be characterized in that it is used as the oxidoreductase.

According to one embodiment, the biosensor may be characterized in that the biosensor comprises: a substrate; an electrode structure provided on the substrate while comprising a working electrode and a reference electrode; and a reagent layer provided on the electrode structure, wherein the reagent layer comprises: an oxidoreductase; and an electron transfer mediator comprising a ruthenium compound and Neutral red.

According to one embodiment, the method of manufacturing a biosensor may be characterized in that the method comprises: providing, on a substrate, an electrode structure comprising a working electrode and a reference electrode; and providing a reagent layer on the electrode structure, wherein the providing of the reagent layer on the electrode structure is a step of providing the reagent layer with an oxidoreductase and an electron transfer mediator comprising a ruthenium compound and Neutral red.

According to one aspect, the providing of the reagent layer may be characterized in that it comprises adjusting the concentration of the Neutral red such that a rate at which a current generated in response to a specimen in the body fluid being subjected to an oxidation-reduction reaction with the oxidoreductase increases in correspondence with an increase in the concentration of the specimen is equal to or greater than a predetermined rate of increase.

According to another aspect, the providing of the reagent layer may be characterized in that it comprises adjusting the concentration of the Neutral red such that a current generated in response to a specimen being subjected to an oxidation-reduction reaction with the oxidoreductase is equal to or greater than a predetermined threshold value.

According to one embodiment, provided is a biosensor for detecting glucose in a body fluid using a measuring device, the biosensor comprising: a substrate; a plurality of electrodes positioned on the substrate; and a reagent layer, wherein the reagent layer comprises: an oxidoreductase; and an electron transfer mediator comprising a ruthenium compound and Neutral red.

According to one aspect, the concentration of the Neutral red may be 0.1 mM or more and 1 mM or less.

According to another aspect, the electrodes may be configured to transmit, to the measuring device, a current signal generated by an oxidation-reduction reaction between the glucose and the reagent layer, and the concentration of the glucose may be determined based on a value of the current signal transmitted to the measuring device.

According to still another aspect, the value of the current signal may vary in proportion to the concentration of the glucose within a numerical range of 357 nA or more and 3601 nA or less, when the concentration of the glucose varies within a numerical range of 40 mg/dL or more and 600 mg/dL or less.

According to still another aspect, the rate of increase of the current signal according to the concentration of the glucose may be 3 nA/(mg/dL) or more and 6 nA/(mg/dL) or less.

According to still another aspect, the ruthenium compound may be at least one compound selected from the group consisting of Hexaammineruthenium trichloride, Ruthenium(III) Hexaamine, Ruthenium(II) Tris(2,2'-bipyridine), Ruthenium(II) Tris(4,4'-dicarboxy-2,2'-bipyridine), and Ruthenium(III) Chloro Di(2,2'-bipyridine).

According to still another aspect, the oxidoreductase may be a glucose dehydrogenase (GDH).

According to still another aspect, the oxidoreductase may be at least one compound selected from the group consisting of FAD-GDH (Flavin Adenine Dinucleotide-dependent Glucose Dehydrogenase), NAD-GDH (Nicotinamide Adenine Dinucleotide-dependent Glucose Dehydrogenase), and PQQ-GDH (Pyrroloquinoline Quinone-dependent Glucose Dehydrogenase).

According to one embodiment, provided is a system for measuring a concentration of glucose in a body fluid, the system comprising: a body fluid collection device; any one of the above-described biosensors; and a measuring device.

According to one embodiment, provided is a method of manufacturing a biosensor for detecting glucose in a body fluid, the method comprising: preparing a substrate; forming a plurality of electrodes on the substrate; and disposing a reagent layer on the electrodes, wherein the reagent layer comprises: an oxidoreductase; and an electron transfer mediator comprising a ruthenium compound and Neutral red.

According to one aspect, the concentration of the Neutral red may be 0.1 mM or more and 1 mM or less.

### EFFECT OF THE INVENTION

By configuring a reagent layer with an oxidoreductase and an electron transfer mediator comprising a ruthenium compound and Neutral red, embodiments may implement current sensing for improving accuracy of specimen measurement.

In this case, embodiments may propose a reagent layer that adjusts a concentration of the electron transfer mediator so as to satisfy a condition that an increase in current corresponding to an increase in the concentration of the specimen in the body fluid is to be equal to or greater than a rate of increase at which sensing is possible, and a condition that the current is to be equal to or greater than a threshold value at which sensing is possible with respect to a reference concentration of the specimen in the body fluid.

However, the effects of the present invention are not limited to the above-described effects, and may be variously expanded within a scope that does not depart from the technical idea and area of the present invention.

### BRIEF DESCRIPTION OF DRAWINGS

FIG. 1 is a diagram illustrating an example of a biosensor system according to one embodiment.
FIG. 2 is a block diagram illustrating an example of a biosensor included in a biosensor system according to one embodiment.
FIG. 3 is a diagram illustrating a structure of Neutral red used as an electron transfer mediator of a reagent layer included in a biosensor according to one embodiment.
FIGS. 4 and 5 are diagrams for explaining superiority in electron transfer of Neutral red used as an electron transfer mediator of a reagent layer included in a biosensor according to one embodiment.
FIG. 6 is a diagram for explaining that a concentration of Neutral red used as an electron transfer mediator of a reagent layer included in a biosensor according to one embodiment is adjusted.
FIG. 7 is a flowchart illustrating a method of manufacturing a biosensor according to one embodiment.

### DETAILED DESCRIPTION

Hereinafter, embodiments of the present invention will be described in detail with reference to the accompanying drawings. However, the present invention is not restricted or limited by the embodiments. In addition, the same reference numerals presented in each drawing denote the same members.

In addition, the terminology used in this specification is terminology used to appropriately express the embodiments of the present invention, and this may vary according to the intention of a viewer or an operator, the custom of the field to which the present invention belongs, or the like. Therefore, definitions of these terms should be made based on the content throughout this specification. For example, in this specification, a singular form also includes a plural form unless specifically stated otherwise in the phrase. In addition, "Comprises" and/or "Comprising" used in this specification does not exclude the presence or addition of one or more other components, steps, operations, and/or elements to the mentioned components, steps, operations, and/or elements. In addition, although the terms such as first and second are used to describe various areas, directions, shapes, and the like in this specification, these areas, directions, and shapes should not be limited by these terms. These terms are used only to distinguish a certain area, direction, or shape from another area, direction, or shape. Therefore, a portion referred to as a first portion in one embodiment may be referred to as a second portion in another embodiment.

In addition, it should be understood that various embodiments of the present invention are different from each other but do not need to be mutually exclusive. For example, a specific shape, structure, and characteristic described herein may be implemented as another embodiment in relation to one embodiment without departing from the technical idea and scope of the present invention. In addition, it should be understood that a position, arrangement, or configuration of an individual component in each presented embodiment category may be changed without departing from the technical idea and scope of the present invention.

Hereinafter, the body fluid may comprise saliva or blood. Specifically, hereinafter, the body fluid may comprise various in-vivo substances such as blood, lymph, plasma, serum, saliva, urine, feces, sweat, mucus, tears, cerebrospinal fluid, nasal secretions, cervical or vaginal secretions, semen, pleural fluid, amniotic fluid, ascites, middle ear fluid, joint fluid, and gastric content extract, but is not limited thereto. In addition, hereinafter, the specimen may comprise glucose. Specifically, hereinafter, the specimen is a target substance to be detected through the biosensor, and may comprise a substance used to measure various physiological indicators and environmental conditions, such as glucose, lactate, ethanol, hemoglobin, cardiolipin, protein, insulin, hormones such as estrogen, microorganisms, DNA/RNA, calcium, sodium, pH, oxygen, and carbon dioxide, but is not limited thereto. In addition, hereinafter, saliva glucose means glucose included in saliva, and blood glucose means glucose included in blood. In addition, hereinafter, acquiring saliva glucose means acquiring information on the saliva glucose (for example, a concentration, an index, a numerical value, or the like of the saliva glucose), and predicting blood glucose means predicting information on the blood glucose (for example, a concentration, an index, a numerical value, or the like of the blood glucose).

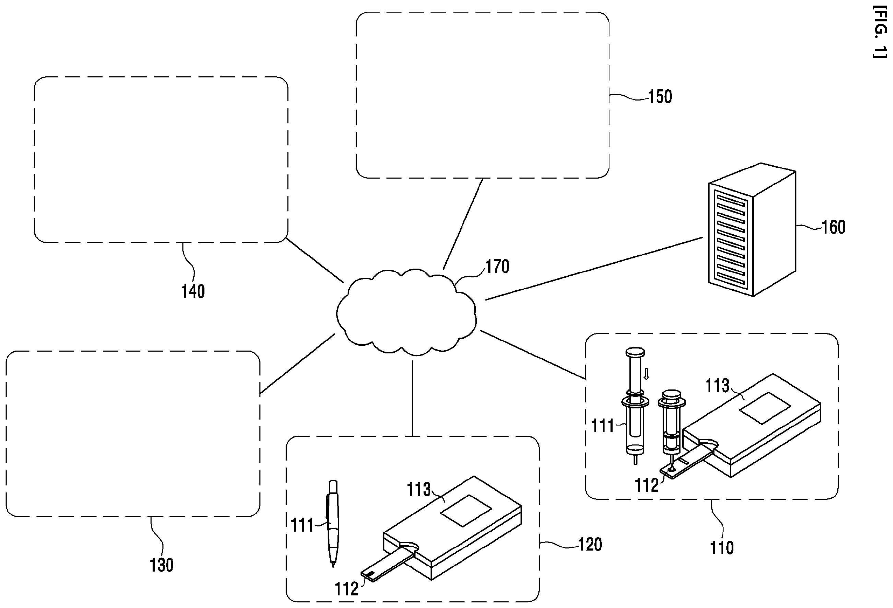

FIG. 1 is a diagram illustrating an example of a biosensor system according to one embodiment. The biosensor system of FIG. 1 illustrates an example comprising a plurality of electronic devices 110, 120, 130, 140, and 150, a server 160, and a network 170. Such FIG. 1 is an example for describing the invention, and the number of electronic devices or the number of servers is not limited as in FIG. 1.

The plurality of electronic devices 110, 120, 130, 140, and 150 are terminals respectively possessed by test subjects, and each of the plurality of electronic devices 110, 120, 130, 140, and 150 may comprise, as a specimen measurement system, a body fluid collection device or a collection device 111, a biosensor 112, and a measuring device 113.

The collection device 111 is a device for collecting a body fluid comprising saliva or blood, and may be a collection tool comprising a specimen collection part, a filter, and a compression tube, or a lancing device comprising a lancet. When the specimen is saliva, the collection device 111 may remove an interfering substance from the collected saliva through the filter within the compression tube, and provide the saliva from which the interfering substance has been removed to the outside, that is, to the biosensor 112. When the specimen is blood, the collection device 111 may form a puncture of a predetermined depth in skin, such as a fingertip edge of a test subject, through the lancet, and provide the blood formed on the skin to the biosensor 112.

The biosensor 112 is a component that performs a function of sensing a specimen to be measured (for example, glucose) when a body fluid comprising saliva or blood is injected into the biosensor 112 through the collection device 111.

When the biosensor 112 is inserted into the measuring device 113, the measuring device 113 recognizes this and provides a specific signal to the biosensor 112, thereby being switched to a standby state for recognizing injection of a body fluid. When a body fluid is injected into the biosensor 112, the measuring device 113 recognizes that the body fluid has been injected through a response signal (for example, a change in resistance or voltage) generated in the biosensor 112. When it is determined that a body fluid has been injected, the measuring device 113 applies a voltage for measuring a specimen (for example, glucose) to the working electrode and the reference electrode. As a result, a response signal (for example, a response current) is generated from the biosensor 112 according to an oxidation-reduction reaction between the specimen in the body fluid and the reagent layer, and the measuring device 113 may receive this and detect the specimen (for example, glucose) or measure or predict a concentration of the specimen.

To this end, the measuring device 113 may comprise a memory, a processor, an input/output interface, and the like.

In addition, the measuring device 113 may comprise a communication module so as to communicate with the server 160 through the network 170. However, the plurality of electronic devices 110, 120, 130, 140, and 150 respectively comprising the measuring device 113 may be configured to communicate, through the network 170, with a terminal possessed by a subject (for example, a smartphone, a mobile phone, a tablet PC, a navigation device, a computer, a laptop, a digital broadcasting terminal, a PDA (Personal Digital Assistant), a PMP (Portable Multimedia Player), or the like), or may be configured to access the network 170 via the terminal and communicate with the server 160, instead of directly accessing the network 170 and communicating with the server 160.

A communication method is not limited, and may include not only a communication method utilizing a communication network (for example, a mobile communication network, a wired Internet, a wireless Internet, and a broadcasting network) that the network 170 may comprise, but also short-range wireless communication between devices. For example, the network 170 may comprise any one or more networks among networks such as a PAN (Personal Area Network), a LAN (Local Area Network), a CAN (Campus Area Network), a MAN (Metropolitan Area Network), a WAN (Wide Area Network), a BBN (Broadband Network), and the Internet. In addition, the network 170 may comprise any one or more of network topologies including a bus network, a star network, a ring network, a mesh network, a star-bus network, a tree or hierarchical network, and the like, but is not limited thereto.

Although it has been described above that each of the plurality of electronic devices 110, 120, 130, 140, and 150 comprises the collection device 111, the biosensor 112, and the measuring device 113, it is not restricted or limited thereto, and a terminal possessed by a test subject (for example, a smartphone, a mobile phone, a tablet PC, a navigation device, a computer, a laptop, a digital broadcasting terminal, a PDA (Personal Digital Assistant), a PMP (Portable Multimedia Player), or the like) may also be comprised as the measuring device 113.

The server 160 may be implemented as a computer device or a plurality of computer devices that communicate with the plurality of electronic devices 110, 120, 130, 140, and 150 through the network 170 to receive specimen measurement information in a body fluid measured from the measuring device 113 of each of the plurality of electronic devices 110, 120, 130, 140, and 150, and that provide a biomarker measurement service for predicting a biomarker (for example, blood glucose) of a subject based on the received specimen measurement information.

That is, each of the plurality of electronic devices 110, 120, 130, 140, and 150 may access the server 160 and be provided with the biomarker measurement service provided by the server 160, under control of a pre-installed operating system (OS) or at least one program (for example, firmware, or a browser or an application installed under the operating system).

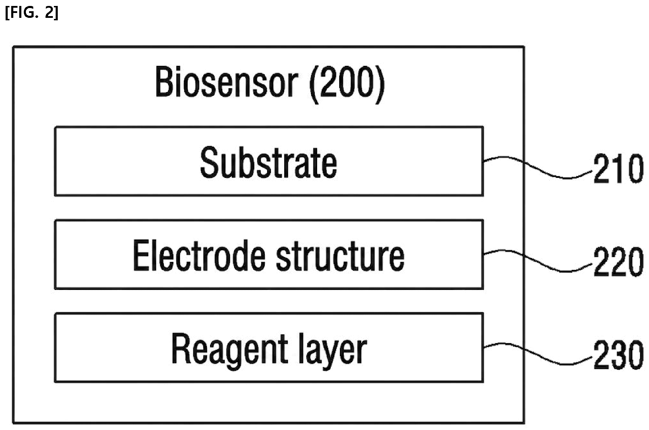

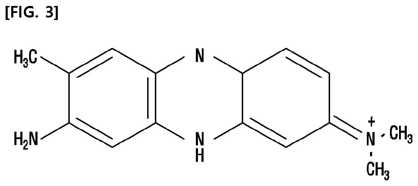

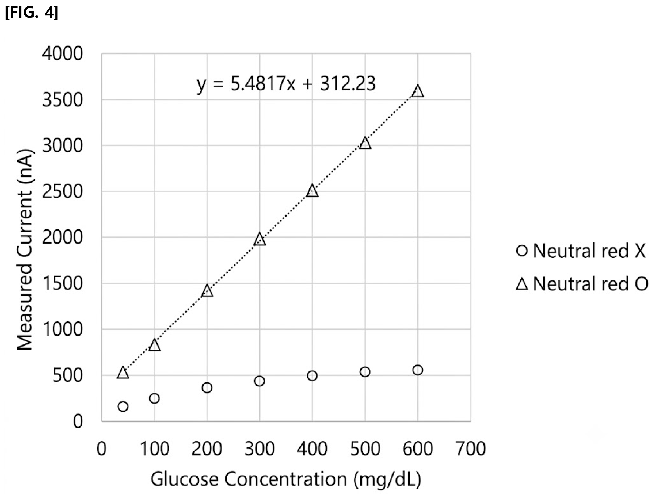

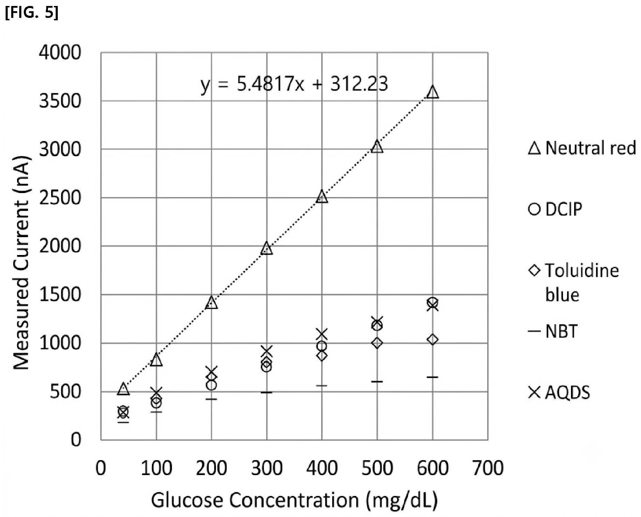

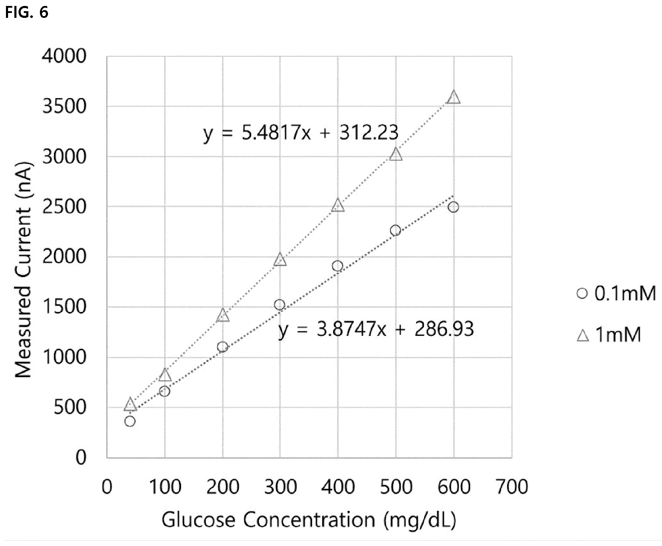

FIG. 2 is a block diagram illustrating an example of a biosensor included in a biosensor system according to one embodiment, FIG. 3 is a diagram illustrating a structure of Neutral red used as an electron transfer mediator of a reagent layer included in a biosensor according to one embodiment, FIGS. 4 and 5 are diagrams for explaining superiority in electron transfer of Neutral red used as an electron transfer mediator of a reagent layer included in a biosensor according to one embodiment, and FIG. 6 is a diagram for explaining that a concentration of Neutral red used as an electron transfer mediator of a reagent layer included in a biosensor according to one embodiment is adjusted.

Referring to FIG. 2, the biosensor 200 may comprise a substrate 210, an electrode structure 220, and a reagent layer 230.

The substrate 210 is a base on which the electrode structure 220 is provided, and may be formed of an insulating material such that the working electrode and the reference electrode included in the electrode structure 220 are electrically separated from each other.

If the working electrode and the reference electrode are each independently formed on planes having different heights instead of being formed on the same plane, the substrate 210 may be composed of a plurality of layers such that the working electrode and the reference electrode are disposed.

The electrode structure 220 may be configured to promote an oxidation-reduction reaction between the specimen in the body fluid and the reagent layer 230 through interaction with the specimen in the body fluid and the reagent layer 230, and to transmit a current signal generated by the oxidation-reduction reaction to the measuring device.

That is, the electrode structure 220 is a component part provided on the substrate 210 while comprising the working electrode and the reference electrode, and through this, a current generated by the reagent layer 230 provided on the working electrode and the reference electrode reacting with the specimen in the body fluid may be measured.

Accordingly, an arrangement structure and a shape of the working electrode and the reference electrode may be variously implemented as long as they satisfy a condition that enables measurement of a current generated by the reagent layer 230 provided on the working electrode and the reference electrode reacting with the specimen in the body fluid.

The reagent layer 230 is provided on the electrode structure 220, and may comprise substances that cause an oxidation-reduction reaction with the specimen in the body fluid injected onto the reagent layer 230. More specifically, the reagent layer 230 may comprise an oxidoreductase and an electron transfer mediator.

The oxidoreductase may comprise a glucose dehydrogenase (GDH). Specifically, the oxidoreductase may comprise at least one compound selected from the group consisting of FAD-GDH (Flavin Adenine Dinucleotide-dependent Glucose Dehydrogenase), NAD-GDH (Nicotinamide Adenine Dinucleotide-dependent Glucose Dehydrogenase), and PQQ-GDH (Pyrroloquinoline Quinone-dependent Glucose Dehydrogenase). However, it is not limited thereto, and the oxidoreductase may comprise various enzymes that cause an oxidation-reduction reaction with the specimen in the body fluid.

The electron transfer mediator may comprise a ruthenium compound. Specifically, the ruthenium compound may comprise at least one compound selected from the group consisting of Hexaammineruthenium trichloride, Ruthenium(III) Hexaamine, Ruthenium(II) Tris(2,2'-bipyridine), Ruthenium(II) Tris(4,4'-dicarboxy-2,2'-bipyridine), and Ruthenium(III) Chloro Di(2,2'-bipyridine). However, it is not limited thereto, and the electron transfer mediator may comprise various compounds that mediate such that electrons generated due to a reaction between the specimen in the body fluid injected onto the reagent layer 230 and the reagent layer 230 comprising the oxidoreductase are transferred to the electrode structure 220 at a high speed.

The electron transfer mediator may further comprise Neutral red. As illustrated in FIG. 3, the Neutral red may have a structure of 3-Amino-7-dimethylamino-2-methylphenazine hydrochloride (C₁₅H₁₇ClN₄).

The graphs illustrated in FIGS. 4 to 6 are respectively based on data described in [Table 1] to [Table 3] below.

**[Table 1]**

| Glucose Concentration (mg/dL) | | 40 | 100 | 200 | 300 | 400 | 500 | 600 |
|---|---|---|---|---|---|---|---|---|
| Neutral red Excluded | Measured Current (nA) | 150.120 | 246.360 | 333.067 | 432.600 | 489.000 | 531.800 | 556.533 |
| | Slope (Increase Rate) | 0.7104 | | | | | | |
| Neutral red Included | Measured Current (nA) | 535.233 | 832.900 | 1420.611 | 1979.056 | 2516.333 | 3031.706 | 3600.588 |
| | Slope (Increase Rate) | 5.4817 | | | | | | |

**[Table 2]**

| Glucose concentration (mg/dL) | | 40 | 100 | 200 | 300 | 400 | 500 | 600 |
|---|---|---|---|---|---|---|---|---|
| DCIP | Measured current (nA) | 294 | 375 | 564 | 750 | 959 | 1178 | 1415 |
| | Slope (Increase Rate) | 2.0065 | | | | | | |
| Toluidine blue | Measured current (nA) | 274 | 425 | 652 | 801 | 870 | 1003 | 1033 |
| | Slope (Increase Rate) | 1.3491 | | | | | | |
| NBT | Measured current (nA) | 170 | 283 | 409 | 491 | 557 | 603 | 646 |
| | Slope (Increase Rate) | 0.8168 | | | | | | |
| AQDS | Measured current (nA) | 283 | 487 | 705 | 913 | 1095 | 1210 | 1389 |
| | Slope (Increase Rate) | 1.9108 | | | | | | |
| Neutral red | Measured current (nA) | 535 | 833 | 1421 | 1979 | 2516 | 3032 | 3601 |
| | Slope (Increase Rate) | 5.4817 | | | | | | |

**[Table 3]**

| Glucose Concentration (mg/dL) | | 40 | 100 | 200 | 300 | 400 | 500 | 600 |
|---|---|---|---|---|---|---|---|---|
| Neutral red 0.1 mM | Measured Current (nA) | 357 | 655 | 1104 | 1522 | 1911 | 2261 | 2490 |
| | Slope (Increase Rate) | 3.8747 | | | | | | |
| Neutral red 1 mM | Measured Current (nA) | 535 | 833 | 1421 | 1979 | 2516 | 3032 | 3601 |
| | Slope (Increase Rate) | 5.4817 | | | | | | |

As such, since the biosensor 200 according to one embodiment uses Neutral red as the electron transfer mediator in the reagent layer 230, as illustrated in FIG. 4, unlike a case where Neutral red is not used as the electron transfer mediator, the biosensor 200 may improve specimen measurement accuracy by satisfying a condition that an increase in current corresponding to an increase in the concentration of the specimen in the body fluid is to be equal to or greater than a rate of increase at which sensing is possible, based on a characteristic that a sensing current sharply increases in correspondence with the concentration of the specimen.

In addition, since the biosensor 200 according to one embodiment uses Neutral red as the electron transfer mediator instead of using an electron transfer mediator such as DCIP (2,6-Dichloro-4-[(4-hydroxyphenyl)imino]cyclohexa-2,5-dien-1-one), Toluidine blue (7-amino-8-methylphenothiazin-3-ylidene)-dimethylammonium chloride), NBT (2,2'-bis(4-Nitrophenyl)-5,5'-diphenyl-3,3'-(3,3'-dimethoxy-4,4'-diphenylene)ditetrazolium chloride), and AQDS (Disodium Anthraquinone-2,6-disulfonate), as illustrated in FIG. 5, the biosensor 200 may have a characteristic of sharply increasing a sensing current in correspondence with the concentration of the specimen, compared to a case of using an electron transfer mediator such as DCIP, Toluidine blue, NBT, and AQDS. Accordingly, the biosensor 200 may improve specimen measurement accuracy by satisfying a condition that an increase in current corresponding to an increase in the concentration of the specimen in the body fluid is to be equal to or greater than a rate of increase at which sensing is possible, based on a characteristic that a sensing current sharply increases in correspondence with the concentration of the specimen.

To this end, the concentration of Neutral red used as the electron transfer mediator may be adjusted such that a rate at which a current generated in response to a specimen being subjected to an oxidation-reduction reaction with the oxidoreductase increases in correspondence with an increase in the concentration of the specimen in the body fluid is equal to or greater than a predetermined rate of increase.

More specifically, a current generated in response to a specimen being subjected to an oxidation-reduction reaction with the oxidoreductase has a characteristic of increasing in proportion to an increase in the concentration of the specimen in the body fluid, and also increasing in proportion to an increase in the concentration of Neutral red. For example, as illustrated in FIG. 6, a current generated in response to a specimen being subjected to an oxidation-reduction reaction with the oxidoreductase increases in proportion to an increase in the concentration of the specimen in the body fluid, and also increases in proportion to an increase in the concentration of Neutral red. Specifically, a current generated in response to a specimen being subjected to an oxidation-reduction reaction with the oxidoreductase has a larger value when the concentration of Neutral red is 1 mM than when it is 0.1 mM.

Accordingly, a rate at which a current generated in response to a specimen being subjected to an oxidation-reduction reaction with the oxidoreductase increases in correspondence with an increase in the concentration of the specimen in the body fluid is increased in proportion to an increase in the concentration of Neutral red. That is, the rate of increase of the current is larger in a case where the concentration of Neutral red is 1 mM than in a case where it is 0.1 mM.

As such, the fact that the rate of increase of the current is larger means ease of current sensing, and thus the concentration of Neutral red may be adjusted such that a rate at which a current generated in response to a specimen being subjected to an oxidation-reduction reaction with the oxidoreductase is increased in correspondence with an increase in the concentration of the specimen in the body fluid is equal to or greater than a predetermined rate of increase (a critical rate of increase at which accurate current sensing is possible). For example, the concentration of Neutral red may be adjusted to 0.1 mM at which the rate of increase of the current is equal to or greater than the predetermined rate of increase (the critical rate of increase at which accurate current sensing is possible). More preferably, the concentration of Neutral red may be adjusted to 1 mM at which the rate of increase of the current is equal to or greater than the predetermined rate of increase (the critical rate of increase at which accurate current sensing is possible).

However, the concentration of Neutral red is not restricted or limited thereto, and may also be adjusted such that a current generated in response to a specimen being subjected to an oxidation-reduction reaction with the oxidoreductase is equal to or greater than a predetermined threshold value (a minimum current value at which current sensing is possible) with respect to a reference concentration of the specimen in the body fluid (a concentration within an average range in which the specimen is included in the body fluid). This corresponds to a minimum condition for the biosensor 200 to detect sensing of a target substance in the specimen. For example, the concentration of Neutral red may be adjusted to 0.1 mM or more and 1 mM or less.

As illustrated in FIG. 6 and described in [Table 3], when the reagent layer comprises Neutral red of 0.1 mM or more and 1 mM or less, the value of a current signal generated in response to a specimen being subjected to an oxidation-reduction reaction with the oxidoreductase may vary in proportion to the concentration of glucose within a numerical range of 357 nA or more and 3601 nA or less, when the concentration of glucose varies within a numerical range of 40 mg/dL or more and 600 mg/dL or less. In this case, the rate of increase of the current signal according to the concentration of glucose may be 3 nA/(mg/dL) or more and 6 nA/(mg/dL) or less.

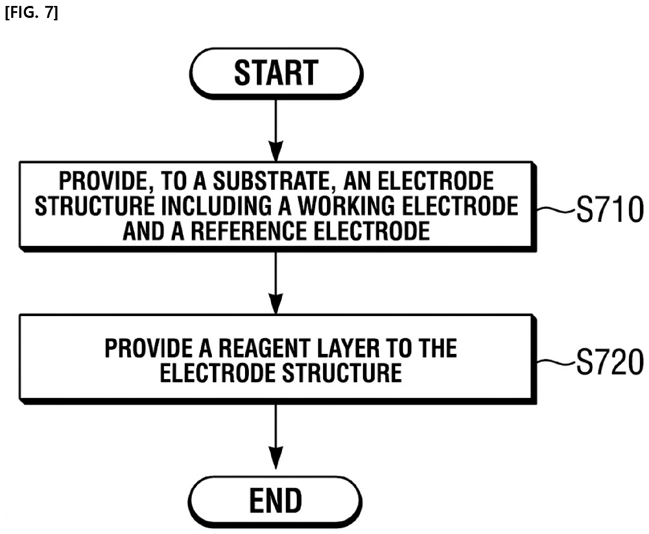

FIG. 7 is a flowchart illustrating a method of manufacturing a biosensor according to one embodiment.

The manufacturing method described below means a process of manufacturing the biosensor described with reference to FIGS. 1 to 6, and is premised on being performed by an automated and mechanized manufacturing system.

Referring to FIG. 7, in a first step S710, the manufacturing system may provide, on a substrate, an electrode structure comprising a working electrode and a reference electrode. Providing, on a substrate, an electrode structure comprising a working electrode and a reference electrode means disposing and forming, on the substrate, the electrode structure comprising the working electrode and the reference electrode.

In a second step S720, the manufacturing system may provide a reagent layer to the electrode structure. Providing a reagent layer to the electrode structure means disposing and forming the reagent layer on the electrode structure.

More specifically, in the second step S720, the manufacturing system may provide a reagent layer comprising an oxidoreductase, and a ruthenium compound and Neutral red as an electron transfer mediator.

The oxidoreductase may comprise a glucose dehydrogenase (GDH). Specifically, the oxidoreductase may comprise at least one compound selected from the group consisting of FAD-GDH (Flavin Adenine Dinucleotide-dependent Glucose Dehydrogenase), NAD-GDH (Nicotinamide Adenine Dinucleotide-dependent Glucose Dehydrogenase), and PQQ-GDH (Pyrroloquinoline Quinone-dependent Glucose Dehydrogenase). However, it is not limited thereto, and the oxidoreductase may comprise various enzymes that cause an oxidation-reduction reaction with the specimen in the body fluid.

The electron transfer mediator may comprise a ruthenium compound. Specifically, the ruthenium compound may be at least one compound selected from the group consisting of Hexaammineruthenium trichloride, Ruthenium(III) Hexaamine, Ruthenium(II) Tris(2,2'-bipyridine), Ruthenium(II) Tris(4,4'-dicarboxy-2,2'-bipyridine), and Ruthenium(III) Chloro Di(2,2'-bipyridine). However, it is not limited thereto, and the electron transfer mediator may comprise various compounds that mediate such that electrons generated due to a reaction between the specimen in the body fluid injected onto the reagent layer and the reagent layer comprising the oxidoreductase are transferred to the electrode structure at a high speed.

The electron transfer mediator may further comprise Neutral red. As illustrated in FIG. 3, the Neutral red may have a structure of 3-Amino-7-dimethylamino-2-methylphenazine hydrochloride (C15H17ClN4).

In particular, in the second step S720, the manufacturing system may enable accurate current sensing by adjusting the concentration of Neutral red such that a rate at which a current generated in response to a specimen being subjected to an oxidation-reduction reaction with the oxidoreductase increases in correspondence with an increase in the concentration of the specimen in the body fluid is equal to or greater than a predetermined rate of increase.

In addition, it is not restricted or limited thereto, and in the second step S720, the manufacturing system may enable current sensing for a specimen at the reference concentration by adjusting the concentration of Neutral red such that a current generated in response to a specimen being subjected to an oxidation-reduction reaction with the oxidoreductase is equal to or greater than a predetermined threshold value with respect to a reference concentration of the specimen in the body fluid.

The device comprising the measuring device 113 described above may be implemented as a hardware component, a software component, and/or a combination of a hardware component and a software component. For example, the devices and components described in the embodiments may be implemented using one or more general-purpose computers or special-purpose computers, such as a processor, a controller, an ALU (Arithmetic Logic Unit), a digital signal processor, a microcomputer, an FPA (Field Programmable Array), a PLU (Programmable Logic Unit), a microprocessor, or any other device capable of executing and responding to an instruction. The processing device may execute an operating system and one or more software applications executed on the operating system. In addition, the processing device may, in response to execution of software, access, store, manipulate, process, and generate data. For convenience of understanding, the processing device has in some cases been described as a single one being used, but a person having ordinary skill in the relevant art may recognize that the processing device may comprise a plurality of processing elements and/or a plurality of types of processing elements. For example, the processing device may comprise a plurality of processors, or one processor and one controller. In addition, another processing configuration, such as a parallel processor, is also possible.

The software may comprise a computer program, code, an instruction, or a combination of one or more of these, and may configure the processing device to operate as desired, or may instruct the processing device independently or collectively. The software and/or data may be embodied in any type of machine, component, physical device, virtual equipment, computer storage medium, or device, in order to be interpreted by the processing device or to provide an instruction or data to the processing device. The software may be distributed over a networked computer system, and stored or executed in a distributed manner. The software and data may be stored in one or more computer-readable recording media.

The method according to the embodiments may be implemented in the form of program instructions that can be performed through various computer means, and recorded on a computer-readable medium. The computer-readable medium may comprise program instructions, data files, data structures, and the like, alone or in combination. The program instructions recorded on the medium may be specially designed and configured for the embodiments, or may be known and usable to those skilled in computer software. Examples of the computer-readable recording medium include magnetic media such as a hard disk, a floppy disk, and a magnetic tape, optical media such as a CD-ROM and a DVD, magneto-optical media such as a floptical disk, and hardware devices specially configured to store and perform program instructions, such as a ROM, a RAM, and a flash memory. Examples of the program instructions include not only machine language code such as that produced by a compiler, but also high-level language code that can be executed by a computer using an interpreter or the like.

Although the embodiments have been described above by the limited embodiments and drawings, various modifications and variations are possible from the above description for a person having ordinary skill in the relevant art. For example, even if the described techniques are performed in an order different from the described method, or the described components such as a system, a structure, a device, and a circuit are coupled or combined in a form different from the described method, or are replaced or substituted by other components or equivalents, appropriate results may be achieved.

Therefore, other implementations, other embodiments, and equivalents to the claims also fall within the scope of the claims described below.

## Claims

1. A biosensor for detecting glucose in a body fluid using a measuring device, comprising:
a substrate;
a plurality of electrodes positioned on the substrate; and
a reagent layer,
wherein the reagent layer comprises:
an oxidoreductase; and
an electron transfer mediator comprising a ruthenium compound and Neutral red.

2. The biosensor of claim 1, wherein a concentration of the Neutral red is 0.1 mM or more and 1 mM or less.

3. The biosensor of claim 1, wherein the electrodes are configured to transmit, to the measuring device, a current signal generated by an oxidation-reduction reaction between the glucose and the reagent layer, and a concentration of the glucose is determined based on a value of the current signal transmitted to the measuring device.

4. The biosensor of claim 3, wherein the value of the current signal varies in proportion to the concentration of the glucose within a numerical range of 357 nA or more and 3601 nA or less, when the concentration of the glucose varies within a numerical range of 40 mg/dL or more and 600 mg/dL or less.

5. The biosensor of claim 3, wherein a rate of increase of the current signal according to the concentration of the glucose is 3 nA/(mg/dL) or more and 6 nA/(mg/dL) or less.

6. The biosensor of claim 1, wherein the ruthenium compound is at least one compound selected from the group consisting of Hexaammineruthenium trichloride, Ruthenium(III) Hexaamine, Ruthenium(II) Tris(2,2'-bipyridine), Ruthenium(II) Tris(4,4'-dicarboxy-2,2'-bipyridine), and Ruthenium(III) Chloro Di(2,2'-bipyridine).

7. The biosensor of claim 1, wherein the oxidoreductase is a glucose dehydrogenase (GDH).

8. The biosensor of claim 7, wherein the oxidoreductase is at least one compound selected from the group consisting of FAD-GDH (Flavin Adenine Dinucleotide-dependent Glucose Dehydrogenase), NAD-GDH (Nicotinamide Adenine Dinucleotide-dependent Glucose Dehydrogenase), and PQQ-GDH (Pyrroloquinoline Quinone-dependent Glucose Dehydrogenase).

9. A system for measuring a concentration of glucose in a body fluid, comprising:
a body fluid collection device;
the biosensor according to any one of claims 1 to 8; and
a measuring device.

10. A method of manufacturing a biosensor for detecting glucose in a body fluid, comprising:
preparing a substrate;
forming a plurality of electrodes on the substrate; and
disposing a reagent layer on the electrodes,
wherein the reagent layer comprises:
an oxidoreductase; and
an electron transfer mediator comprising a ruthenium compound and Neutral red.

11. The method of claim 10, wherein a concentration of the Neutral red is 0.1 mM or more and 1 mM or less.
